(19) Europäisches Patentamt
European Patent Office
Office européen des brevets

(11) **EP 4 140 963 A1**

(12) **EUROPÄISCHE PATENTANMELDUNG**

(43) Veröffentlichungstag:
**01.03.2023 Patentblatt 2023/09**

(21) Anmeldenummer: **21192546.6**

(22) Anmeldetag: **23.08.2021**

(51) Internationale Patentklassifikation (IPC):
**C03C 3/097** (2006.01) **C03C 4/00** (2006.01)
**C03C 10/00** (2006.01)

(52) Gemeinsame Patentklassifikation (CPC):
**C03C 3/097; C03C 4/0021; C03C 10/0009**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Ivoclar Vivadent AG**
**9494 Schaan (LI)**

(72) Erfinder: **Ritzberger, Christian**
**9472 Grabs (CH)**

(74) Vertreter: **Uexküll & Stolberg**
**Partnerschaft von**
**Patent- und Rechtsanwälten mbB**
**Beselerstraße 4**
**22607 Hamburg (DE)**

(54) **LITHIUMSILIKAT-GLASKERAMIK MIT LEICHTER BEARBEITBARKEIT**

(57) Die Erfindung betrifft Lithiumsilikat-Glaskeramik, die Lithiummetasilikat als Hauptkristallphase aufweist und die folgenden Komponenten in den angegebenen Mengen enthält:

| Komponente | Gew.-% |
|---|---|
| $SiO_2$ | 71,0 bis 82,0 |
| $Li_2O$ | 6,0 bis 14,0 |
| $Me^I_2O$ | 4,0 bis 15,0 |
| $Al_2O_3$ | 2,0 bis 10,0 |
| $P_2O_5$ | 0,5 bis 7,0, |

wobei $Me^I_2O$ ausgewählt ist aus $Na_2O$, $K_2O$, $Rb_2O$, $CS_2O$ und Mischungen davon und wobei das molare Verhältnis von $SiO_2$ zu $Li_2O$ im Bereich von 2,5 bis 5,0 liegt.

EP 4 140 963 A1

**Beschreibung**

Die Erfindung betrifft Lithiumsilikat-Glaskeramik, die sich vor allem zum Einsatz in der Zahnheilkunde und insbesondere zur Herstellung von dentalen Restaurationen eignet, sowie Vorstufen zur Herstellung dieser Glaskeramik.

Lithiumsilikat-Glaskeramiken zeichnen sich in der Regel durch sehr gute mechanische Eigenschaften aus, weshalb sie seit einiger Zeit im Dentalbereich und dort vornehmlich zur Herstellung von Dentalkronen und kleinen Dentalbrücken Anwendung finden.

WO 95/32678 A2 beschreibt Lithiumdisilikat-Glaskeramiken, die durch Verpressen im viskosen Zustand zu Dentalrestaurationen verarbeitet werden. Dabei ist allerdings die Verwendung eines deformierbaren Tiegels zwingend erforderlich, was die Verarbeitung sehr aufwändig gestaltet.

EP 0 827 941 A1 und EP 0 916 625 A1 offenbaren Lithiumdisilikat-Glaskeramiken, denen durch Verpressen oder maschinelle Bearbeitung die Form der gewünschten Dentalrestauration gegeben werden kann.

EP 1 505 041 A1 und die EP 1 688 398 A1 beschreiben Verfahren zur Herstellung von Dentalrestaurationen aus Lithiumdisilikat-Glaskeramiken. Dabei wird zunächst als Zwischenstufe eine Glaskeramik mit Lithiummetasilikat als Hauptkristallphase erzeugt, die sich mechanisch z.B. mittels CAD/CAM-Verfahren, bearbeiten lässt. Diese Zwischenstufe wird dann einer weiteren Wärmebehandlung unterzogen, um die gewünschte hochfeste Lithiumdisilikat-Glaskeramik zu bilden.

Die maschinelle Bearbeitung der konventionellen Lithiumdisilikat-Glaskeramiken ist aufgrund ihrer hohen Festigkeit nur schwierig möglich und geht daher regelmäßig mit einem hohen Verschleiß der eingesetzten Werkzeuge einher. Die maschinelle Bearbeitung von Lithiummetasilikat-Glaskeramiken ist grundsätzlich leichter und mit weniger Werkzeugverschleiß möglich. Allerdings lassen sich die bekannten Lithiummetasilikat-Glaskeramiken nur relativ langsam maschinell, beispielsweise durch die Schleifkörper üblicher CAD/CAM-Maschinen, bearbeiten. Dies ist vor allem für die vielfach gewünschte Versorgung eines Patienten mit einer dentalen Restauration in einer einzigen Behandlungssitzung (sogenannte Chairside-Behandlung) problematisch.

Es besteht daher ein Bedarf an Lithiumsilikat-Glaskeramiken, die sich im Vergleich zu den bekannten Lithiummetasilikat-Glaskeramiken schneller maschinell bearbeiten lassen und anschließend zu hochfesten Dentalprodukten umgewandelt werden können, die zudem eine hohe chemische Beständigkeit und ausgezeichnete optische Eigenschaften zeigen.

Diese Aufgabe wird durch die Lithiumsilikat-Glaskeramik nach den Ansprüchen 1 bis 10 und 12 gelöst. Gegenstand der Erfindung sind ebenfalls das Ausgangsglas nach den Ansprüchen 11 und 12, das Verfahren nach den Ansprüchen 13 und 14, die Verwendung nach den Ansprüchen 15 bis 17 sowie das Verfahren nach Anspruch 18.

Die erfindungsgemäße Lithiumsilikat-Glaskeramik zeichnet sich dadurch aus, dass sie Lithiummetasilikat als Hauptkristallphase aufweist und die folgenden Komponenten in den angegebenen Mengen enthält:

| Komponente | Gew.-% |
|---|---|
| $SiO_2$ | 71,0 bis 82,0 |
| $Li_2O$ | 6,0 bis 14,0 |
| $Me^I_2O$ | 4,0 bis 15,0 |
| $Al_2O_3$ | 2,0 bis 10,0 |
| $P_2O_5$ | 0,5 bis 7,0, |

wobei $Me^I_2O$ ausgewählt ist aus $Na_2O$, $K_2O$, $Rb_2O$, $Cs_2O$ und Mischungen davon und wobei das molare Verhältnis von $SiO_2$ zu $Li_2O$ im Bereich von 2,5 bis 5,0 liegt.

Es hat sich überraschenderweise gezeigt, dass die erfindungsgemäße Glaskeramik eine Kombination von sehr wünschenswerten mechanischen und optischen Eigenschaften in sich vereinigt, wie sie gerade für ein restauratives Dentalmaterial erforderlich sind. Diese Glaskeramik hat eine niedrige Festigkeit und Zähigkeit und kann demgemäß leicht und in sehr kurzer Zeit durch maschinelle Bearbeitung in die Form sogar von komplizierten Dentalrestaurationen gebracht werden, kann aber nach einer solchen maschinellen Bearbeitung mit Hilfe einer Wärmebehandlung in ein Glaskeramikprodukt mit hervorragenden mechanischen Eigenschaften, ausgezeichneten optischen Eigenschaften und sehr guter chemischer Stabilität umgewandelt werden.

Mit dem Begriff "Hauptkristallphase" wird die Kristallphase bezeichnet, die von allen in der Glaskeramik vorhandenen Kristallphasen den höchsten Massenanteil hat. Die Bestimmung der Massen der Kristallphasen erfolgt dabei insbesondere mit der Rietveld-Methode. Ein geeignetes Verfahren zur quantitativen Analyse der Kristallphasen mittels der Rietveld-Methode ist z.B. in der Dissertation von M. Dittmer "Gläser und Glaskeramiken im System $MgO-Al_2O_3-SiO_2$ mit $ZrO_2$ als Keimbildner", Universität Jena 2011, beschrieben.

Die erfindungsgemäße Lithiumsilikat-Glaskeramik enthält insbesondere 73,1 bis 80,0 und bevorzugt 74,0 bis 78,0 Gew.-% $SiO_2$.

Es ist weiter bevorzugt, dass die Glaskeramik 7,0 bis 12,9 und bevorzugt 8,0 bis 12,0 Gew.-% $Li_2O$ enthält. Es wird angenommen, dass $Li_2O$ die Viskosität der Glasmatrix erniedrigt und damit die Kristallisation der gewünschten Phasen fördert.

In einer weiteren bevorzugten Ausführungsform enthält die Glaskeramik 5,1 bis 10,0 und bevorzugt 5,5 bis 7,0 Gew.-% weiteres Oxid einwertiger Elemente $Me^I_2O$, wobei $Me^I_2O$ aus $Na_2O$, $K_2O$, $Rb_2O$, $Cs_2O$ und Mischungen davon ausgewählt ist und vorzugsweise $K_2O$ ist.

Besonders bevorzugt enthält die Glaskeramik mindestens eines und insbesondere alle der folgenden weiteren Oxide einwertiger Elemente $Me^I_2O$ in den angegebenen Mengen:

| Komponente | Gew.-% |
|---|---|
| $Na_2O$ | 0 bis 2,0 |
| $K_2O$ | 0 bis 10,0 |
| $Rb_2O$ | 0 bis 13,0 |
| $Cs_2O$ | 0 bis 13,0. |

In einer besonders bevorzugten Ausführungsform enthält die erfindungsgemäße Glaskeramik 2,0 bis 10,0, bevorzugt 5,1 bis 9,0 und besonders bevorzugt 5,5 bis 7,0 Gew.-% $K_2O$.

Auch ist es bevorzugt, dass die Glaskeramik 4,0 bis 7,0 und bevorzugt 5,1 bis 6,5 Gew.-% $Al_2O_3$ enthält.

In einer weiteren bevorzugten Ausführungsform enthält die Glaskeramik 1,0 bis 4,0, bevorzugt 1,2 bis 2,6 und besonders bevorzugt 1,5 bis 2,5 Gew.-% $P_2O_5$. Es wird angenommen, dass das $P_2O_5$ als Keimbildner wirkt.

Des Weiteren ist es bevorzugt, dass die Glaskeramik 1,0 bis 9,0, bevorzugt 2,0 bis 8,0 und besonders bevorzugt 3,0 bis 7,0 Gew.-% Oxid zweiwertiger Elemente $Me^{II}O$ ausgewählt aus der Gruppe von MgO, CaO, SrO, ZnO und Mischungen davon enthält.

In einer weiteren bevorzugten Ausführungsform enthält die Glaskeramik weniger als 2,0 Gew.-% an BaO. Die Glaskeramik ist insbesondere im Wesentlichen frei von BaO.

Bevorzugt enthält die Glaskeramik mindestens eines und insbesondere alle der folgenden Oxide zweiwertiger Elemente $Me^{II}O$ in den angegebenen Mengen:

| Komponente | Gew.-% |
|---|---|
| MgO | 0 bis 4,0 |
| CaO | 0 bis 4,0 |
| SrO | 0 bis 7,0 |
| ZnO | 0 bis 5,0. |

In einer besonders bevorzugten Ausführungsform enthält die Glaskeramik 0,1 bis 4,0, bevorzugt 0,5 bis 3,0 und besonders bevorzugt 1,0 bis 2,0 Gew.-% MgO.

In einer weiteren besonders bevorzugten Ausführungsform enthält die Glaskeramik 0,1 bis 7,0, bevorzugt 1,0 bis 6,0, besonders bevorzugt 2,0 bis 5,0 und ganz besonders bevorzugt 3,0 bis 4,0 Gew.-% SrO.

Es ist weiter eine Glaskeramik bevorzugt, die 0 bis 8,0, bevorzugt 1,0 bis 7,0 und besonders bevorzugt 2,0 bis 6,0 Gew.-% weiteres Oxid dreiwertiger Elemente $Me^{III}_2O_3$ ausgewählt aus der Gruppe von $B_2O_3$, $Y_2O_3$, $La_2O_3$, $Ga_2O_3$, $In_2O_3$ und Mischungen davon enthält.

Besonders bevorzugt enthält die Glaskeramik mindestens eines und insbesondere alle der folgenden weiteren Oxide dreiwertiger Elemente $Me^{III}_2O_3$ in den angegebenen Mengen:

| Komponente | Gew.-% |
|---|---|
| $B_2O_3$ | 0 bis 4,0 |
| $Y_2O_3$ | 0 bis 5,0 |
| $La_2O_3$ | 0 bis 5,0 |
| $Ga_2O_3$ | 0 bis 3,0 |
| $In_2O_3$ | 0 bis 5,0. |

Ferner ist eine Glaskeramik bevorzugt, die 0 bis 10,0, bevorzugt 1,0 bis 8,0 und besonders bevorzugt 2,0 bis 6,0 Gew.-% Oxid vierwertiger Elemente $Me^{IV}O_2$ ausgewählt aus der Gruppe von $TiO_2$, $ZrO_2$, $GeO_2$, $SnO_2$, $CeO_2$ und Mischungen davon enthält.

Besonders bevorzugt enthält die Glaskeramik mindestens eines und insbesondere alle der folgenden Oxide vierwertiger Elemente $Me^{IV}O_2$ in den angegebenen Mengen:

| Komponente | Gew.-% |
|---|---|
| $TiO_2$ | 0 bis 4,0 |
| $ZrO_2$ | 0 bis 3,0 |
| $GeO_2$ | 0 bis 9,0 |
| $SnO_2$ | 0 bis 3,0 |
| $CeO_2$ | 0 bis 4,0. |

In einer weiteren Ausführungsform enthält die Glaskeramik 0 bis 8,0, bevorzugt 1,0 bis 7,0 und besonders bevorzugt 2,0 bis 6,0 Gew.-% weiteres Oxid fünfwertiger Elemente $Me^{V}_2O_5$ ausgewählt aus der Gruppe von $V_2O_5$, $Nb_2O_5$, $Ta_2O_5$ und Mischungen davon.

Besonders bevorzugt enthält die Glaskeramik mindestens eines und insbesondere alle der folgenden weiteren Oxide fünfwertiger Elemente $Me^{V}_2O_5$ in den angegebenen Mengen:

| Komponente | Gew.-% |
|---|---|
| $V_2O_5$ | 0 bis 2,0 |
| $Nb_2O_5$ | 0 bis 5,0 |
| $Ta_2O_5$ | 0 bis 5,0. |

In einer weiteren Ausführungsform enthält die Glaskeramik 0 bis 5,0, bevorzugt 1,0 bis 4,0 und besonders bevorzugt 2,0 bis 3,0 Gew.-% Oxid sechswertiger Element $Me^{VI}O_3$ ausgewählt aus der Gruppe von $MoO_3$, $WO_3$ und Mischungen davon.

Besonders bevorzugt enthält die Glaskeramik mindestens eines und insbesondere alle der folgenden Oxide $Me^{VI}O_3$ in den angegebenen Mengen:

| Komponente | Gew.-% |
|---|---|
| $MoO_3$ | 0 bis 3,0 |
| $WO_3$ | 0 bis 3,0. |

In einer weiteren Ausführungsform enthält die erfindungsgemäße Glaskeramik 0 bis 1,0 und insbesondere 0 bis 0,5 Gew.-% Fluor.

Besonders bevorzugt ist eine Glaskeramik, die mindestens eine und bevorzugt alle der folgenden Komponenten in den angegebenen Mengen enthält:

| Komponente | Gew.-% |
|---|---|
| $SiO_2$ | 73,1 bis 80,0 |
| $Li_2O$ | 7,0 bis 12,9 |
| $Me^{I}_2O$ | 4,0 bis 15,0, insbesondere 5,1 bis 10,0 |
| $Al_2O_3$ | 4,0 bis 10,0 |
| $P_2O_5$ | 1,2 bis 2,6 |
| $Me^{II}O$ | 0 bis 9,0 |
| $Me^{III}_2O_3$ | 0 bis 8,0 |
| $Me^{IV}O_2$ | 0 bis 10,0 |
| $Me^{V}_2O_5$ | 0 bis 8,0 |
| $Me^{VI}O_3$ | 0 bis 5,0 |
| Fluor | 0 bis 1,0, |

wobei $Me^{I}_2O$, $Me^{II}O$, $Me^{III}_2O_3$, $Me^{IV}O_2$, $Me^{V}_2O_5$ und $Me^{VI}O_3$ die oben angegebenen Bedeutungen haben.

In einer weiteren besonders bevorzugten Ausführungsform enthält die Glaskeramik mindestens eine und bevorzugt alle der folgenden Komponenten in den angegebenen Mengen:

| Komponente | Gew.-% |
|---|---|
| $SiO_2$ | 73,1 bis 80,0 |
| $Li_2O$ | 7,0 bis 12,9 |
| $Al_2O_3$ | 4,0 bis 10,0 |
| $P_2O_5$ | 1,2 bis 2,6 |
| $Na_2O$ | 0 bis 2,0 |
| $K_2O$ | 0 bis 10,0 |
| $Rb_2O$ | 0 bis 13,0 |
| $Cs_2O$ | 0 bis 13,0 |
| MgO | 0 bis 4,0 |
| CaO | 0 bis 4,0 |
| SrO | 0 bis 7,0 |
| ZnO | 0 bis 5,0 |
| $B_2O_3$ | 0 bis 4,0 |
| $Y_2O_3$ | 0 bis 5,0 |
| $La_2O_3$ | 0 bis 5,0 |
| $Ga_2O_3$ | 0 bis 3,0 |
| $In_2O_3$ | 0 bis 5,0 |
| $TiO_2$ | 0 bis 4,0 |
| $ZrO_2$ | 0 bis 3,0 |
| $GeO_2$ | 0 bis 9,0 |
| $SnO_2$ | 0 bis 3,0 |
| $CeO_2$ | 0 bis 4,0 |
| $V_2O_5$ | 0 bis 2,0 |
| $Nb_2O_5$ | 0 bis 5,0 |
| $Ta_2O_5$ | 0 bis 5,0 |
| $MoO_3$ | 0 bis 3,0 |
| $WO_3$ | 0 bis 3,0 |
| Fluor | 0 bis 1,0. |

Manche der vorstehend genannten Komponenten können als Färbemittel und/oder Fluoreszenzmittel dienen. Die erfindungsgemäße Glaskeramik kann darüber hinaus noch weitere Färbemittel und/oder Fluoreszenzmittel enthalten. Diese können z.B. aus $Bi_2O_3$ oder $Bi_2O_5$ und insbesondere aus weiteren anorganischen Pigmenten und/oder Oxiden von d- und f-Elementen, wie z.B. den Oxiden von Mn, Fe, Co, Pr, Nd, Tb, Er, Dy, Eu und Yb, ausgewählt sein. Mithilfe dieser Färbemittel und Fluoreszenzmittel ist eine einfache Einfärbung der Glaskeramik möglich, um die gewünschten optischen Eigenschaften insbesondere von natürlichem Zahnmaterial zu imitieren.

In einer bevorzugten Ausführungsform der Glaskeramik liegt das molare Verhältnis von $SiO_2$ zu $Li_2O$ im Bereich von 2,9 bis 4,6 und bevorzugt im Bereich von 3,3 bis 4,4. Es ist überraschend, dass trotz dieser hohen molaren Überschüsse an $SiO_2$ relativ zu $Li_2O$ die erfindungsgemäßen Glaskeramiken mit Lithiummetasilikat als Hauptkristallphase gebildet werden können.

Vorzugsweise enthält die erfindungsgemäße Glaskeramik nicht mehr als 30 Gew.-%, bevorzugt nicht mehr als 28 Gew.-%, besonders bevorzugt nicht mehr als 26 Gew.-% und ganz besonders bevorzugt nicht mehr als 22 Gew.-% an Lithiummetasilikatkristallen. Besonders bevorzugt enthält die Glaskeramik 10 bis 30 Gew.-%, bevorzugt 12 bis 28 Gew.-%, besonders bevorzugt 15 bis 26 Gew.-% und ganz besonders bevorzugt 18 bis 22 Gew.-% an Lithiummetasilikatkristallen.

Es ist weiter bevorzugt, dass in der erfindungsgemäßen Glaskeramik die mittlere Größe der Lithiummetasilikatkristalle im Bereich von 5 bis 80 nm, insbesondere im Bereich von 10 bis 50 nm, bevorzugt im Bereich von 15 bis 45 nm und besonders bevorzugt im Bereich von 25 bis 35 nm liegt.

Die mittlere Größe der Lithiummetasilikatkristalle kann insbesondere anhand einer Röntgenbeugung an einem Pulver der Glaskeramik mit $CuK_\alpha$-Strahlung bestimmt werden. Das dabei erhaltene Röntgendiffraktogramm kann dazu nach der Rietveld-Methode ausgewertet und aus der Halbwertsbreite der Peaks von Lithiummetasilikat nach der Scherrer-Gleichung die mittlere Größe der Lithiummetasilikatkristalle berechnet werden. Diese Auswertung kann vorzugsweise softwaregestützt erfolgen, beispielsweise mit der Software TOPAS 5.0 der Firma Bruker.

Die erfindungsgemäße Glaskeramik kann zusätzlich zu Lithiummetasilikat noch weitere Kristallphasen, wie z.B. Quarz, insbesondere Tiefquarz, Apatit, Cäsiumalumosilikat und insbesondere Lithiumphosphat enthalten. Dabei sollte die Menge an Cristobalit jedoch möglichst klein sein und insbesondere weniger als 1,0 Gew.-% betragen. Es ist besonders bevorzugt, dass die erfindungsgemäße Glaskeramik im Wesentlichen frei von Cristobalit ist.

Die Art und insbesondere die Menge der gebildeten Kristallphasen können durch die Zusammensetzung des Ausgangsglases sowie die Wärmebehandlung gesteuert werden, die zur Herstellung der Glaskeramik aus dem Ausgangsglas angewendet wird. Die Beispiele veranschaulichen dies anhand der Variation der Zusammensetzung des Ausgangsglases und der angewendeten Wärmebehandlung.

Die Glaskeramik weist eine biaxiale Bruchfestigkeit von vorzugsweise mindestens 80 MPa und besonders bevorzugt 100 bis 200 MPa auf. Die biaxiale Bruchfestigkeit wurde gemäß ISO 6872 (2008) (Kolbenauf-drei-Kugeln-Prüfung) bestimmt.

Die erfindungsgemäße Glaskeramik weist einen thermischen Ausdehnungskoeffizienten WAK (gemessen im Bereich von 100 bis 500°C) von bevorzugt 9,5 bis $14,0 \cdot 10^{-6}$ $K^{-1}$ auf. Der WAK wird gemäß ISO 6872 (2008) bestimmt. Eine Einstellung des Wärmeausdehnungskoeffizienten auf einen gewünschten Wert erfolgt insbesondere durch die Art und Menge der in der Glaskeramik vorhandenen Kristallphasen sowie die chemische Zusammensetzung der Glaskeramik.

Die Transluzenz der Glaskeramik wurde in Form des Kontrastwerts (CR-Wert) gemäß British Standard BS 5612 bestimmt, und dieser Kontrastwert betrug vorzugsweise 40 bis 92.

Die Erfindung betrifft ebenfalls verschiedene Vorstufen mit entsprechender Zusammensetzung, aus denen die erfindungsgemäße Lithiumsilikat-Glaskeramik durch Wärmebehandlung hergestellt werden kann. Diese Vorstufen sind ein entsprechend zusammengesetztes Ausgangsglas und ein entsprechend zusammengesetztes Ausgangsglas mit Keimen. Die Bezeichnung "entsprechende Zusammensetzung" bedeutet, dass diese Vorstufen die gleichen Komponenten in den gleichen Mengen wie die Glaskeramik enthalten, wobei die Komponenten mit Ausnahme von Fluor als Oxide berechnet werden, so wie es bei Gläsern und Glaskeramiken üblich ist.

Die Erfindung betrifft daher ebenfalls ein Ausgangsglas, das die Komponenten der erfindungsgemäßen Lithiumsilikat-Glaskeramik enthält.

Das erfindungsgemäße Ausgangsglas enthält daher insbesondere geeignete Mengen an $SiO_2$, $Li_2O$, $Me^I_2O$, $Al_2O_3$ und $P_2O_5$, die zur Ausbildung der erfindungsgemäßen Glaskeramik mit Lithiummetasilikat als Hauptkristallphase erforderlich sind. Weiter kann das Ausgangsglas auch noch andere Komponenten enthalten, wie sie oben für die erfindungsgemäße Lithiumsilikat-Glaskeramik angegeben sind. Es sind alle solche Ausführungsformen für die Komponenten des Ausgangsglases bevorzugt, die auch für die Komponenten der erfindungsgemäßen Lithiumsilikat-Glaskeramik als bevorzugt angegeben sind.

Die Erfindung betrifft ebenfalls ein solches Ausgangsglas, das Keime für die Bildung von Lithiummetasilikatkristallen enthält.

Die erfindungsgemäße Lithiumsilikat-Glaskeramik und das erfindungsgemäße Ausgangsglas liegen insbesondere in Form von Pulvern, Granulaten oder Rohlingen in beliebiger Form und Größe, z.B. monolithischen Rohlingen, wie Plättchen, Quadern oder Zylindern, oder Pulverpresslingen, in ungesinterter, teilgesinterter oder dichtgesinterter Form, vor. In diesen Formen können sie einfach weiterverarbeitet werden. Sie können aber auch in Form von dentalen Restaurationen, wie Inlays, Onlays, Kronen, Veneers, Schalen oder Abutments, vorliegen.

Zur Herstellung des Ausgangsglases wird insbesondere so vorgegangen, dass eine Mischung von geeigneten Ausgangsmaterialien, wie z.B. Carbonaten, Oxiden, Phosphaten und Fluoriden, bei Temperaturen von insbesondere 1300 bis 1600°C für 2 bis 10 h erschmolzen wird. Zur Erzielung einer besonders hohen Homogenität wird die erhaltene Glasschmelze in Wasser gegossen, um ein Glasgranulat zu bilden, und das erhaltene Granulat wird dann erneut aufgeschmolzen.

Die Schmelze kann dann in Formen gegossen werden, um Rohlinge des Ausgangsglases, sogenannte Massivglasrohlinge oder monolithische Rohlinge, zu erzeugen.

Es ist ebenfalls möglich, die Schmelze erneut in Wasser zu geben, um ein Granulat herzustellen. Dieses Granulat kann nach Mahlen und gegebenenfalls Zugabe weiterer Komponenten, wie Färbe- und Fluoreszenzmitteln, zu einem Rohling, einem sogenannten Pulverpressling, gepresst werden.

Schließlich kann das Ausgangsglas nach Granulierung auch zu einem Pulver verarbeitet werden.

Anschließend wird das Ausgangsglas, z.B. in Form eines Massivglasrohlings, eines Pulverpresslings oder in Form eines Pulvers, mindestens einer Wärmebehandlung unterzogen. Es ist bevorzugt, dass zunächst eine erste Wärmebehandlung durchgeführt wird, um das erfindungsgemäße Ausgangsglas mit Keimen für die Bildung von Lithiummetasilikatkristallen herzustellen. Das Ausgangsglas mit Keimen wird dann üblicherweise mindestens einer weiteren Temperaturbehandlung bei einer höheren Temperatur unterworfen, um Kristallisation von Lithiummetasilikat zu bewirken und die erfindungsgemäße Lithiumsilikat-Glaskeramik herzustellen.

Die Erfindung betrifft somit ebenfalls ein Verfahren zur Herstellung der erfindungsgemäßen Lithiumsilikat-Glaskeramik, bei dem das Ausgangsglas oder das Ausgangsglas mit Keimen mindestens einer Wärmebehandlung bei einer Temperatur von 450 bis 750°C für eine Dauer von insbesondere 1 bis 120 min, bevorzugt 5 bis 120 min und besonders bevorzugt 10 bis 60 min, unterzogen wird.

Die im erfindungsgemäßen Verfahren durchgeführte mindestens eine Wärmebehandlung kann auch im Rahmen eines Heißpressens oder Aufsinterns des erfindungsgemäßen Ausgangsglases oder des erfindungsgemäßen Ausgangsglases mit Keimen erfolgen.

Es ist bevorzugt, das Ausgangsglas einer Wärmebehandlung bei einer Temperatur von 450 bis 600°C, bevorzugt 480 bis 580°C und besonders bevorzugt 480 bis 520°C, für eine Dauer von insbesondere 1 bis 120 min, bevorzugt 10 bis 60 min, zu unterwerfen, um das Ausgangsglas mit Keimen für die Kristallisation von Lithiummetasilikat zu erzeugen.

Es ist weiter bevorzugt, das Ausgangsglas mit Keimen einer Wärmebehandlung bei einer Temperatur von 550 bis 750°C, bevorzugt 580 bis 700°C und besonders bevorzugt 590 bis 630°C, für eine Dauer von insbesondere 1 bis 120 min, bevorzugt 5 bis 60 min und besonders bevorzugt 10 bis 30 min, zu unterwerfen, um die Lithiumsilikat-Glaskeramik herzustellen.

In einer bevorzugten Ausführungsform umfasst somit das erfindungsgemäße Verfahren zur Herstellung der Lithiumsilikat-Glaskeramik, dass

(a) das Ausgangsglas einer Wärmebehandlung bei einer Temperatur von 450 bis 600°C, bevorzugt 480 bis 580°C und besonders bevorzugt 480 bis 520°C, für eine Dauer von insbesondere 1 bis 120 min, bevorzugt 10 bis 60 min, unterworfen wird, um Ausgangsglas mit Keimen zu bilden, und

(b) das Ausgangsglas mit Keimen einer Wärmebehandlung bei einer Temperatur von 550 bis 750°C, bevorzugt 580 bis 700°C und besonders bevorzugt 590 bis 630°C, für eine Dauer von insbesondere 1 bis 120 min, bevorzugt 5 bis 60 min und besonders bevorzugt 10 bis 30 min, unterworfen wird, um die Glaskeramik zu bilden.

Aufgrund der vorstehend geschilderten Eigenschaften der erfindungsgemäßen Glaskeramiken und der erfindungsgemäßen Gläser eignen sich diese insbesondere zum Einsatz in der Zahnheilkunde. Gegenstand der Erfindung ist daher auch die Verwendung der erfindungsgemäßen Glaskeramiken oder der erfindungsgemäßen Gläser als Dentalmaterial und insbesondere zur Herstellung dentaler Restaurationen oder als Beschichtungsmaterial für dentale Restaurationen. Insbesondere können aus den erfindungsgemäßen Glaskeramiken und den erfindungsgemäßen Gläsern dentale Restaurationen, wie Brücken, Inlays, Onlays, Veneers, Abutments, Teilkronen, Kronen oder Schalen, hergestellt werden. Die Erfindung betrifft daher auch die Verwendung der erfindungsgemäßen Glaskeramiken oder der erfindungsgemäßen Gläser zur Herstellung dentaler Restaurationen. Dabei ist es bevorzugt, dass der Glaskeramik oder dem Glas durch Verpressen oder maschinelle Bearbeitung die Form der gewünschten dentalen Restauration gegeben wird.

Die Erfindung betrifft auch ein Verfahren zur Herstellung von dentalen Restaurationen, bei dem den erfindungsgemäßen Glaskeramiken oder den erfindungsgemäßen Gläsern durch Verpressen oder maschinelle Bearbeitung die Form der gewünschten dentalen Restauration gegeben wird.

Das Verpressen erfolgt üblicherweise unter erhöhtem Druck und erhöhter Temperatur. Es ist bevorzugt, dass das Verpressen bei einer Temperatur von 700 bis 1200°C erfolgt. Weiter ist es bevorzugt, das Verpressen bei einem Druck von 2 bis 10 bar durchzuführen. Beim Verpressen wird durch viskoses Fließen des eingesetzten Materials die gewünschte Formänderung erreicht. Es können für das Verpressen das erfindungsgemäße Ausgangsglas und insbesondere das erfindungsgemäße Ausgangsglas mit Keimen, und die erfindungsgemäße Lithiumsilikat-Glaskeramik verwendet werden. Dabei können die erfindungsgemäßen Gläser und Glaskeramiken insbesondere in Form von Rohlingen in beliebiger Form und Größe, z.B. Massivrohlingen oder Pulverpresslingen, z.B. in ungesinterter, teilgesinterter oder dichtgesinterter Form, eingesetzt werden.

Die maschinelle Bearbeitung erfolgt üblicherweise durch materialabtragende Verfahren und insbesondere durch Fräsen und/oder Schleifen. Es ist besonders bevorzugt, dass die maschinelle Bearbeitung im Rahmen eines CAD/CAM-Verfahrens durchgeführt wird. Für die maschinelle Bearbeitung können das erfindungsgemäße Ausgangsglas, das erfindungsgemäße Ausgangsglas mit Keimen und die erfindungsgemäße Lithiumsilikat-Glaskeramik verwendet werden. Dabei können die erfindungsgemäßen Gläser und Glaskeramiken insbesondere in Form von Rohlingen, z.B. Massivrohlingen oder Pulverpresslingen, z.B. in ungesinterter, teilgesinterter oder dichtgesinterter Form, eingesetzt werden. Für die maschinelle Bearbeitung wird bevorzugt die erfindungsgemäße Lithiumsilikat-Glaskeramik verwendet.

Es hat sich überraschend gezeigt, dass sich die erfindungsgemäßen Lithiumsilikat-Glaskeramiken bei gleicher Krafteinwirkung schneller als bekannte Lithiumsilikat-Glaskeramiken bearbeiten lassen. Zur Beschreibung dieser Eigenschaft kann insbesondere die Abtragrate an Probenkörpern der Glaskeramiken bestimmt werden. Hierzu werden von den Probenkörpern Plättchen abgesägt und gewogen. Anschließend werden die Plättchen auf einen Halter geklebt und unter Wasserkühlung mit einer automatischen Schleifmaschine, wie sie beispielsweise von der Fa. Struers erhältlich ist, mittels einer Diamantschleifscheibe, beispielsweise mit einer Körnung von 20 μm, beschliffen. Der Druck der Schleifmaschine wird dabei so gewählt, dass auf jedes Plättchen dieselbe Kraft, beispielsweise 15 N, einwirkt. Nachdem die Plättchen 1 min lang beschliffen wurden, werden diese getrocknet und erneut gewogen. Die Abtragrate wird dann gemäß der folgenden Formel berechnet:

$$\text{Abtragrate } [\text{Gew.-}\% \cdot \text{min}^{-1}] = 100 \times (1 - (m_{\text{beschliffen}} : m_{\text{unbeschliffen}}))$$

Es hat sich weiter gezeigt, dass die leicht maschinell bearbeitbare Lithiumsilikat-Glaskeramik mit Lithiummetasilikat als Hauptkristallphase durch eine weitere Wärmebehandlung zu einer Glaskeramik mit Lithiumdisilikat als Hauptkristallphase umgewandelt werden kann. Diese Glaskeramik weist nicht nur ausgezeichnete mechanische Eigenschaften wie etwa hohe Festigkeit auf, sondern zeigt auch andere Eigenschaften, die für ein Material für Dentalrestaurationen erforderlich sind.

Nachdem die Glaskeramik die Form der gewünschten dentalen Restauration erhalten hat, kann diese somit einer weiteren Wärmebehandlung unterworfen werden, um Lithiummetasilikatkristalle in Lithiumdisilikatkristalle umzuwandeln. Vorzugsweise wird dabei die Glaskeramik einer Wärmebehandlung bei einer Temperatur von 750 bis 950°C, bevorzugt 820 bis 890°C und besonders bevorzugt 840 bis 870°C, insbesondere für eine Dauer von 1 bis 60 Minuten, bevorzugt 5 bis 30 Minuten, besonders bevorzugt 5 bis 15 Minuten und ganz besonders bevorzugt 5 bis 10 Minuten, unterworfen. Die geeigneten Bedingungen für eine gegebene Glaskeramik können beispielsweise bestimmt werden, indem Röntgenbeugungsanalysen bei unterschiedlichen Temperaturen durchgeführt werden.

Es hat sich auch gezeigt, dass die Umwandlung zu einer Lithiumdisilikat-Glaskeramik lediglich mit einer sehr geringen linearen Schrumpfung von nur etwa 0,2 bis 0,3% verbunden ist, was im Vergleich zu einer linearen Schrumpfung von bis zu 30% bei der Sinterung von Keramiken fast vernachlässigbar ist.

Die erfindungsgemäßen Glaskeramiken und die erfindungsgemäßen Gläser eignen sich allerdings auch als Beschichtungsmaterial von z.B. Keramiken und Glaskeramiken. Die Erfindung ist daher ebenfalls auf die Verwendung der erfindungsgemäßen Gläser oder der erfindungsgemäßen Glaskeramiken zur Beschichtung von Keramiken, Glaskeramiken und insbesondere von dentalen Restaurationen gerichtet.

Die Erfindung betrifft auch ein Verfahren zur Beschichtung von Keramiken, Metallen, Metalllegierungen und Glaskeramiken, bei dem erfindungsgemäße Glaskeramik oder erfindungsgemäßes Glas auf die Keramik oder Glaskeramik aufgebracht und erhöhter Temperatur ausgesetzt wird.

Dies kann insbesondere durch Aufsintern oder durch Fügen eines mittels CAD-CAM hergestellten Überwurfs mit einem geeigneten Glaslot oder Kleber und bevorzugt durch Aufpressen erfolgen. Beim Aufsintern wird die Glaskeramik oder das Glas in üblicher Weise, z.B. als Pulver, auf das zu beschichtende Material, wie Keramik oder Glaskeramik, aufgebracht und anschließend bei erhöhter Temperatur gesintert. Bei dem bevorzugten Aufpressen wird erfindungsgemäße Glaskeramik oder erfindungsgemäßes Glas, z.B. in Form von Pulverpresslingen oder monolithischen Rohlingen, bei einer erhöhten Temperatur, von z.B. 700 bis 1200°C, und unter Anwendung von Druck, z.B. 2 bis 10 bar, aufgepresst. Hierzu können insbesondere die in der EP 231 773 beschriebenen Verfahren und der dort offenbarte Pressofen eingesetzt werden. Ein geeigneter Ofen ist z.B. der Programat EP 5000 von Ivoclar Vivadent AG, Liechtenstein.

Es ist bevorzugt, dass nach Abschluss des Beschichtungsvorganges eine Glaskeramik mit Lithiumsilikat, insbesondere Lithiumdisilikat, als Hauptkristallphase vorliegt, da eine solche Glaskeramik über besonders gute Eigenschaften verfügt.

Die Erfindung wird im Folgenden anhand von sie nicht-beschränkenden Beispielen näher erläutert.

Beispiele

Es wurden insgesamt 28 erfindungsgemäße Gläser sowie ein Vergleichsbeispiel mit den in der Tabelle I angegebenen Zusammensetzungen hergestellt. Die Gläser wurden gemäß Tabelle II zu Glaskeramiken kristallisiert. Dabei bedeuten

| | |
|---|---|
| $T_g$ | Glasübergangstemperatur, bestimmt mittels DSC |
| $T_S$ und $t_S$ | angewendete Temperatur und Zeit zum Erschmelzen |
| $T_{Kb}$ und $t_{Kb}$ | angewendete Temperatur und Zeit für Keimbildung |
| $T_{C1}$ und $t_{C1}$ | angewendete Temperatur und Zeit für 1. Kristallisation |
| $T_{C2}$ und $t_{C2}$ | angewendete Temperatur und Zeit für 2. Kristallisation |

In den Beispielen wurden zunächst Ausgangsgläser mit den in der Tabelle I angegebenen Zusammensetzungen im 100 bis 200 g Maßstab aus üblichen Rohstoffen bei der Temperatur $T_S$ für die Dauer $t_S$ erschmolzen, wobei das Erschmelzen sehr gut ohne Bildung von Blasen oder Schlieren möglich war. Durch Eingießen der Ausgangsgläser in Wasser wurden Glasfritten hergestellt, die zur Homogenisierung anschließend ein zweites Mal bei 1500°C oder 1400°C für 1 Stunde geschmolzen wurden. Die erhaltenen Schmelzen des Ausgangsglases wurden anschließend in eine Graphitform gegossen, um Massivglasblöcke zu erzeugen.

Eine erste Wärmebehandlung der so erhaltenen Glasmonolithe bei der Temperatur $T_{Kb}$ für die Dauer $t_{Kb}$ führte zur Bildung von Gläsern mit Keimen. Diese keimhaltigen Gläser kristallisierten durch eine weitere Wärmebehandlung bei der Temperatur $T_{C1}$ für die Dauer $t_{C1}$ zu Glaskeramiken mit Lithiummetasilikat als Hauptkristallphase, wie durch Röntgenbeugungsuntersuchungen bei Raumtemperatur festgestellt wurde.

Die Mengen der Kristallphasen und die mittlere Größe der Lithiummetasilikatkristalle wurden mittels Röntgenbeugung bestimmt. Dazu wurden mittels Mahlen und Sieben (< 45 μm) Pulver der jeweiligen Glaskeramik hergestellt und mit $Al_2O_3$ (Alfa Aesar, Produkt-Nr. 42571) als internem Standard im Verhältnis von 80 Gew.-% Glaskeramik zu 20 Gew.-% $Al_2O_3$ gemischt wurden. Diese Mischung wurde mit Aceton aufgeschlämmt, um eine möglichst gute Durchmischung zu erreichen. Anschließend wurde die Mischung bei etwa 80°C getrocknet. Danach wurde mittels eines D8 Advance Diffraktometers der Firma Bruker ein Diffraktogramm im Bereich 10 bis 100° 2θ mittels CuKα-Strahlung und einer Schrittweite von 0,014° 2θ aufgenommen. Dieses Diffraktogramm wurde dann mit der Software TOPAS 5.0 der Firma Bruker nach der der Rietveld-Methode ausgewertet. Durch Vergleich der Intensitäten der Peaks von Lithiummetasilikat bzw. $Al_2O_3$ wurden die Phasenanteile bestimmt. Aus der Halbwertsbreite der Peaks von Lithiummetasilikat wurde nach der Scherrer-Gleichung die mittlere Größe der Lithiummetasilikatkristalle bestimmt.

Zur Bestimmung der mechanischen Bearbeitbarkeit wurden von den so erhaltenen Glaskeramikblöcken je zwei Plättchen mit einer Fläche von 170 mm$^2$ ± 10 mm$^2$ (ca. 12.5 mm × 13.8 mm) und einer Dicke von 4,0 ±0,5 mm abgesägt und auf einer Präzisionswaage gewogen. Anschließend wurden die Plättchen auf einen Halter geklebt und unter Wasserkühlung mit einer automatischen Schleifmaschine (LaboForche-100, Fa. Struers) mittels einer Diamantschleifscheibe mit einer Körnung von 20 μm beschliffen. Der Druck der Schleifmaschine wurde dabei so gewählt, dass auf jedes Plättchen eine Kraft von 15 N einwirkte. Der Drehteller, auf dem die Diamantschleifscheibe befestigt war, und der Kopf der Schleifmaschine, auf dem die Halter mit den Proben befestigt ist, wiesen die gleiche Drehrichtung auf. Die Geschwindigkeit des Drehtellers betrug 300 U min$^{-1}$. Die Plättchen wurden 1 min lang beschliffen und anschließend getrocknet und erneut gewogenen. Die Abtragrate wurde gemäß der folgenden Formel berechnet:

$$\text{Abtragrate } [\text{Gew.-\% } \cdot \text{min}^{-1}] = 100 \times (1 - (m_{\text{beschliffen}} : m_{\text{unbeschliffen}}))$$

Wie aus Tabelle II ersichtlich ist, waren die Abtragraten bei den erfindungsgemäßen Beispielen 1 bis 28 durchgehend höher als im Vergleichsbeispiel. Dies zeigt, dass sich die erfindungsgemäßen Lithiumsilikat-Glaskeramiken bei gleicher Krafteinwirkung schneller als bekannte Lithiumsilikat-Glaskeramiken bearbeiten lassen.

Die verbliebenen Glaskeramikblöcke wurden einer weiteren Wärmebehandlung bei der Temperatur $T_{C2}$ für die Dauer $t_{C2}$ unterworfen. Dadurch wurden Glaskeramiken mit Lithiumdisilikat als Hauptkristallphase gebildet. Als Nebenphasen wurden Lithiumphosphat sowie bei Beispiel 26 zusätzlich Lithiumstrontiumphosphat gefunden.

Tabelle I

| Beispiel | 1 | 2 | 3 | 4 | 5 | 6 | 7 | 8 | 9 | 10 |
|---|---|---|---|---|---|---|---|---|---|---|
| Zusammen setzung | Gew.-% | Gew.-% | Gew.-% | Gew.-% | Gew.-% | Gew.-% | Gew.-% | Gew.-% | Gew.-% | Gew.-% |
| SiO₂ | 75,9 | 77,1 | 76,9 | 76,6 | 76,9 | 77,3 | 77,6 | 77,8 | 78,2 | 75,3 |
| Li₂O | 11,5 | 10,4 | 10,3 | 10,3 | 10,1 | 9,8 | 9,6 | 9,2 | 8,8 | 10,7 |
| Na₂O | - | - | - | - | - | - | - | - | - | - |
| K₂O | 5,5 | 4,7 | 5,6 | 5,8 | 5,8 | 5,8 | 5,8 | 5,9 | 6,0 | 5,5 |
| Rb₂O | - | - | - | - | - | - | - | - | - | - |
| MgO | - | - | - | - | - | - | - | - | - | - |
| CaO | - | - | - | - | - | - | - | - | - | - |
| SrO | - | - | - | - | - | - | - | - | - | - |
| ZnO | - | - | - | - | - | - | - | - | - | - |
| Al₂O₃ | 4,6 | 5,5 | 4,9 | 5,0 | 5,0 | 5,0 | 5,0 | 5,3 | 5,3 | 4,8 |
| Y₂O₃ | - | - | - | - | - | - | - | - | - | - |
| La₂O₃ | - | - | - | - | - | - | - | - | - | - |
| TiO₂ | - | - | - | - | - | - | - | - | - | - |
| ZrO₂ | - | - | - | - | - | - | - | - | - | - |
| CeO₂ | - | - | - | - | - | - | - | - | - | - |
| P₂O₅ | 2,5 | 2,3 | 2,3 | 2,3 | 2,2 | 2,1 | 2,0 | 1,8 | 1,7 | 2,3 |
| V₂O₅ | - | - | - | - | - | - | - | - | - | - |
| Ta₂O₅ | - | - | - | - | - | - | - | - | - | 1,4 |
| Er₂O₃ | - | - | - | - | - | - | - | - | - | - |
| Tb₄O₇ | - | - | - | - | - | - | - | - | - | - |
| Σ | 100,0 | 100,0 | 100,0 | 100,0 | 100,0 | 100,0 | 100,0 | 100,0 | 100,0 | 100,0 |

| Beispiel | 11 | 12 | 13 | 14 | 15 | 16 | 17 | 18 | 19 | 20 |
|---|---|---|---|---|---|---|---|---|---|---|
| Zusammen setzung | Gew.-% | Gew.-% | Gew.-% | Gew.-% | Gew.-% | Gew.-% | Gew.-% | Gew.-% | Gew.-% | Gew.-% |
| $SiO_2$ | 74,9 | 74,9 | 76,4 | 75,5 | 74,6 | 74,8 | 77,5 | 76,9 | 76,2 | 74,0 |
| $Li_2O$ | 10,3 | 11,0 | 9,7 | 9,6 | 9,5 | 9,5 | 8,4 | 10,1 | 9,0 | 9,2 |
| $Na_2O$ | – | – | – | – | – | – | – | 0,8 | – | – |
| $K_2O$ | 5,5 | 5,5 | 6,8 | 7,8 | 8,8 | 6,8 | 6,4 | 5,0 | 5,9 | 5,8 |
| $Rb_2O$ | – | – | – | – | – | – | – | – | – | – |
| $MgO$ | – | – | – | – | – | – | – | – | – | – |
| $CaO$ | – | – | – | – | – | – | – | – | – | – |
| $SrO$ | – | – | – | – | – | – | – | – | – | – |
| $ZnO$ | – | – | – | – | – | – | – | – | – | – |
| $Al_2O_3$ | 4,8 | 4,7 | 5,0 | 5,0 | 5,0 | 6,8 | 6,1 | 5,0 | 5,3 | 5,0 |
| $Y_2O_3$ | – | 1,4 | – | – | – | – | – | – | – | – |
| $La_2O_3$ | 2,1 | – | – | – | – | – | – | – | – | – |
| $TiO_2$ | – | – | – | – | – | – | – | – | 0,6 | – |
| $ZrO_2$ | – | – | – | – | – | – | – | – | – | 4,0 |
| $CeO_2$ | – | – | – | – | – | – | – | – | 1,2 | – |
| $P_2O_5$ | 2,4 | 2,5 | 2,1 | 2,1 | 2,1 | 2,1 | 1,6 | 2,2 | 1,8 | 2,0 |
| $V_2O_5$ | – | – | – | – | – | – | – | – | – | – |
| $Ta_2O_5$ | – | – | – | – | – | – | – | – | – | – |
| $Er_2O_3$ | – | – | – | – | – | – | – | – | – | – |
| $Tb_4O_7$ | – | – | – | – | – | – | – | – | – | – |
| $\sum$ | 100,0 | 100,0 | 100,0 | 100,0 | 100,0 | 100,0 | 100,0 | 100,0 | 100,0 | 100,0 |

| Beispiel | 21 | 22 | 23 | 24 | 25 | 26 | 27 | 28 | V* |
|---|---|---|---|---|---|---|---|---|---|
| Zusammensetzung | Gew.-% | Gew.-% | Gew.-% | Gew.-% | Gew.-% | Gew.-% | Gew.-% | Gew.-% | Gew.-% |
| $SiO_2$ | 75,4 | 73,1 | 74,3 | 74,5 | 75,5 | 76,3 | 74,4 | 75,1 | 71,7 |
| $Li_2O$ | 8,9 | 7,9 | 9,7 | 9,7 | 9,6 | 8,2 | 9,3 | 12,9 | 14,9 |
| $Na_2O$ | – | – | 0,8 | – | – | – | – | – | – |
| $K_2O$ | 2,9 | – | 4,8 | 5,8 | 5,8 | 6,4 | 5,8 | 5,5 | 4,0 |
| $Rb_2O$ | 5,6 | 12,0 | 3,2 | – | – | – | – | – | – |
| $MgO$ | – | – | – | – | – | 1,4 | – | – | – |
| $CaO$ | – | – | – | – | 2 | – | – | – | – |
| $SrO$ | – | – | – | – | – | – | 3,5 | – | – |
| $ZnO$ | – | – | – | 2,8 | – | – | – | – | – |
| $Al_2O_3$ | 5,3 | 5,4 | 5,0 | 5,0 | 5,0 | 6,1 | 5,0 | 3,6 | 3,3 |
| $Y_2O_3$ | – | – | – | – | – | – | – | – | – |
| $La_2O_3$ | – | – | – | – | – | – | – | – | – |
| $TiO_2$ | – | – | – | – | – | – | – | – | – |
| $ZrO_2$ | – | – | – | – | – | – | – | – | 0,4 |
| $CeO_2$ | – | – | – | – | – | – | – | – | 1,8 |
| $P_2O_5$ | 1,9 | 1,6 | 2,2 | 2,2 | 2,1 | 1,6 | 2,0 | 2,9 | 3,2 |
| $V_2O_5$ | – | – | – | – | – | – | – | – | 0,1 |
| $Ta_2O_5$ | – | – | – | – | – | – | – | – | – |
| $Er_2O_3$ | – | – | – | – | – | – | – | – | 0,1 |
| $Tb_4O_7$ | – | – | – | – | – | – | – | – | 0,5 |
| $\sum$ | 100,0 | 100,0 | 100,0 | 100,0 | 100,0 | 100,0 | 100,0 | 100,0 | 100,0 |

*Vergleich

Tabelle II

| Beispiel | 1 | 2 | 3 | 4 | 5 | 6 | 7 | 8 | 9 | 10 |
|---|---|---|---|---|---|---|---|---|---|---|
| $T_g$ [°C] | 477 | 474 | 476 | 475 | 481 | 477 | 479 | 486 | 486 | 478 |
| $T_S$ [°C] | 1550 | 1500 | 1500 | 1500 | 1550 | 1550 | 1550 | 1600 | 1600 | 1600 |
| $t_S$ [min] | 60 | 60 | 60 | 60 | 60 | 60 | 60 | 60 | 60 | 60 |
| $T_{Kb}$ [°C] | 500 | 500 | 500 | 500 | 500 | 500 | 500 | 500 | 500 | 500 |
| $T_{Kb}$ [min] | 10 | 10 | 10 | 10 | 10 | 10 | 10 | 10 | 10 | 10 |
| $T_{C1}$ [°C] | 610 | 590 | 600 | 580 | 590 | 580 | 600 | 610 | 600 | 700 |
| $t_{C1}$ [min] | 30 | 20 | 5 | 30 | 10 | 30 | 10 | 50 | 10 | 30 |
| Hauptkris-tallphase | $Li_2SiO_3$ | $Li_2SiO_3$ | $Li_2SiO_3$ | $Li_2SiO_3$ | $Li_2SiO_3$ | $Li_2SiO_3$ | $Li_2SiO_3$ | $Li_2SiO_3$ | $Li_2SiO_3$ | $Li_2SiO_3$ |
| Phasenanteil [Gew.-%] | | | | | | | | 20 | | |
| Kristallit-größe [nm] | | | | | | | | 31 | | |
| Abtragrate [Gew.-%·min⁻¹] | 52,4 | 50,6 | 47,2 | 44,2 | 50,4 | 42,6 | 44,9 | 41,4 | 45,1 | 47,6 |
| $T_{C2}$ [°C] | 840 | 850 | 850 | 850 | 850 | 850 | 850 | 850 | 850 | 850 |
| $t_{C2}$ [min] | 7 | 7 | 7 | 7 | 7 | 7 | 7 | 7 | 7 | 7 |
| Kristall-phase(n) | $Li_2Si_2O_5$ $Li_3PO_4$ | $Li_2Si_2O_5$ $Li_3PO_4$ | $Li_2Si_2O_5$ $Li_3PO_4$ | $Li_2Si_2O_5$ $Li_3PO_4$ | $Li_2Si_2O_5$ $Li_3PO_4$ | $Li_2Si_2O_5$ $Li_3PO_4$ | $Li_2Si_2O_5$ $Li_3PO_4$ | $Li_2Si_2O_5$ $Li_3PO_4$ | $Li_2Si_2O_5$ $Li_3PO_4$ | $Li_2Si_2O_5$ $Li_3PO_4$ |

| Beispiel | 11 | 12 | 13 | 14 | 15 | 16 | 17 | 18 | 19 | 20 |
|---|---|---|---|---|---|---|---|---|---|---|
| $T_g$ [°C] | 483 | 476 | 472 | 477 | 473 | 482 | 488 | 474 | 491 | 501 |
| $T_S$ [°C] | 1500 | 1500 | 1550 | 1550 | 1550 | 1550 | 1600 | 1600 | 1500 | 1550 |
| $t_S$ [min] | 60 | 60 | 60 | 60 | 60 | 60 | 60 | 60 | 60 | 60 |
| $T_{Kb}$ [°C] | 500 | 500 | 500 | 500 | 500 | 500 | 500 | 500 | 500 | 500 |
| $T_{Kb}$ [min] | 10 | 10 | 10 | 10 | 10 | 10 | 10 | 10 | 10 | 10 |
| $T_{C1}$ [°C] | 620 | 620 | 630 | 590 | 630 | 610 | 610 | 630 | 630 | 650 |
| $t_{C1}$ [min] | 30 | 30 | 30 | 10 | 30 | 30 | 10 | 30 | 30 | 20 |
| Hauptkristallphase | $Li_2SiO_3$ | $Li_2SiO_3$ | $Li_2SiO_3$ | $Li_2SiO_3$ | $Li_2SiO_3$ | $Li_2SiO_3$ | $Li_2SiO_3$ | $Li_2SiO_3$ | $Li_2SiO_3$ | $Li_2SiO_3$ |
| Phasenanteil [Gew.-%] | | | | | 19 | 20 | 16 | | 19 | |
| Kristallitgröße [nm] | | | | | 37 | 24 | 26 | | 27 | |
| Abtragrate [Gew.-%·min⁻¹] | 44,4 | 41,8 | 47,0 | 48,8 | 38,8 | 45,4 | 43,7 | 38,5 | 55,7 | 49,7 |
| $T_{C2}$ [°C] | 840 | 840 | 840 | 840 | 840 | 860 | 860 | 840 | 840 | 850 |
| $t_{C2}$ [min] | 7 | 7 | 7 | 7 | 7 | 7 | 7 | 7 | 7 | 7 |
| Kristallphase(n) | $Li_2Si_2O_5$ $Li_3PO_4$ | $Li_2Si_2O_5$ $Li_3PO_4$ | $Li_2Si_2O_5$ $Li_3PO_4$ | $Li_2Si_2O_5$ $Li_3PO_4$ | $Li_2Si_2O_5$ $Li_3PO_4$ | $Li_2Si_2O_5$ $Li_3PO_4$ | $Li_2Si_2O_5$ $Li_3PO_4$ | $Li_2Si_2O_5$ $Li_3PO_4$ | $Li_2Si_2O_5$ $Li_3PO_4$ | $Li_2Si_2O_5$ $Li_3PO_4$ |

EP 4 140 963 A1

| Beispiel | 21 | 22 | 23 | 24 | 25 | 26 | 27 | 28 | V* |
|---|---|---|---|---|---|---|---|---|---|
| $T_g$ [°C] | 490 | 497 | 472 | 474 | 479 | 485 | 475 | 469 | |
| $T_s$ [°C] | 1500 | 1500 | 1500 | 1500 | 1500 | 1550 | 1500 | 1500 | |
| $t_s$ [min] | 60 | 60 | 60 | 60 | 60 | 60 | 60 | 60 | |
| $T_{Kb}$ [°C] | 510 | 490 | 530 | 520 | 500 | 480 | 520 | 580 | 500 |
| $T_{Kb}$ [min] | 30 | 50 | 10 | 3 | 10 | 60 | 20 | 40 | 10 |
| $T_{c1}$ [°C] | 630 | 600 | 620 | 600 | 630 | 630 | 620 | 590 | 680 |
| $t_{c1}$ [min] | 30 | 30 | 30 | 30 | 30 | 30 | 30 | 20 | 20 |
| Hauptkristallphase | $Li_2SiO_3$ | $Li_2SiO_3$ | $Li_2SiO_3$ | $Li_2SiO_3$ | $Li_2SiO_3$ | $Li_2SiO_3$ | $Li_2SiO_3$ | $Li_2SiO_3$ | $Li_2SiO_3$ |
| Phasenanteil [Gew.-%] | 20 | 15 | 19 | 22 | 22 | 18 | 22 | 26 | 34 |
| Kristallitgröße [nm] | 24 | 24 | 34 | 12 | 38 | 26 | 34 | 45 | 46 |
| Abtragrate [Gew.-%·min$^{-1}$] | 44,9 | 44,9 | 45,1 | 47,1 | 45,3 | 51,2 | 61,9 | 45,2 | 36,6 |
| $T_{c2}$ [°C] | 870 | 890 | 840 | 840 | 830 | 840 | 820 | 850 | 850 |
| $t_{c2}$ [min] | 7 | 7 | 7 | 7 | 7 | 7 | 7 | 15 | 7 |
| Kristallphase(n) | $Li_2Si_2O_5$ $Li_3PO_4$ | $Li_2Si_2O_5$ $Li_3PO_4$ | $Li_2Si_2O_5$ $Li_3PO_4$ | $Li_2Si_2O_5$ $Li_3PO_4$ | $Li_2Si_2O_5$ $Li_3PO_4$ | $Li_2Si_2O_5$ $LiSrPO_4$ $Li_3PO_4$ | $Li_2Si_2O_5$ $Li_3PO_4$ | $Li_2Si_2O_5$ $Li_3PO_4$ | $Li_2Si_2O_5$ $Li_3PO_4$ |

*Vergleich

## Patentansprüche

1.  Lithiumsilikat-Glaskeramik, die Lithiummetasilikat als Hauptkristallphase aufweist und die folgenden Komponenten in den angegebenen Mengen enthält:

| Komponente | Gew.-% |
|---|---|
| $SiO_2$ | 71,0 bis 82,0 |
| $Li_2O$ | 6,0 bis 14,0 |
| $Me^I_2O$ | 4,0 bis 15,0 |
| $Al_2O_3$ | 2,0 bis 10,0 |
| $P_2O_5$ | 0,5 bis 7,0, |

wobei $Me^I_2O$ ausgewählt ist aus $Na_2O$, $K_2O$, $Rb_2O$, $Cs_2O$ und Mischungen davon und wobei das molare Verhältnis von $SiO_2$ zu $Li_2O$ im Bereich von 2,5 bis 5,0 liegt.

2. Glaskeramik nach Anspruch 1, die 73,1 bis 80,0 und bevorzugt 74,0 bis 78,0 Gew.-% $SiO_2$ enthält.

3. Glaskeramik nach Anspruch 1 oder 2, die 7,0 bis 12,9 und bevorzugt 8,0 bis 12,0 Gew.-% $Li_2O$ enthält.

4. Glaskeramik nach einem der Ansprüche 1 bis 3, die 5,1 bis 10,0 und bevorzugt 5,5 bis 7,0 Gew.-% weiteres Oxid einwertiger Elemente $Me^I_2O$ enthält, wobei $Me^I_2O$ aus $Na_2O$, $K_2O$, $Rb_2O$, $Cs_2O$ und Mischungen davon ausgewählt ist und vorzugsweise $K_2O$ ist.

5. Glaskeramik nach einem der Ansprüche 1 bis 4, die 4,0 bis 7,0 und bevorzugt 5,1 bis 6,5 Gew.-% $Al_2O_3$ enthält.

6. Glaskeramik nach einem der Ansprüche 1 bis 5, die 1,0 bis 4,0, bevorzugt 1,2 bis 2,6 und besonders bevorzugt 1,5 bis 2,5 Gew.-% $P_2O_5$ enthält.

7. Glaskeramik nach einem der Ansprüche 1 bis 6, die mindestens eine und bevorzugt alle der folgenden Komponenten in den angegebenen Mengen enthalten:

| Komponente | Gew.-% |
|---|---|
| $SiO_2$ | 73,1 bis 80,0 |
| $Li_2O$ | 7,0 bis 12,9 |
| $Me^I_2O$ | 4,0 bis 15,0, insbesondere 5,1 bis 10,0 |
| $Al_2O_3$ | 4,0 bis 10,0 |
| $P_2O_5$ | 1,2 bis 2,6 |
| $Me^{II}O$ | 0 bis 9,0 |
| $Me^{III}_2O_3$ | 0 bis 8,0 |
| $Me^{IV}O_2$ | 0 bis 10,0 |
| $Me^V_2O_5$ | 0 bis 8,0 |
| $Me^{VI}O_3$ | 0 bis 5,0 |
| Fluor | 0 bis 1,0, |

wobei

$Me^I_2O$ ausgewählt ist aus $Na_2O$, $K_2O$, $Rb_2O$, $Cs_2O$ und Mischungen davon,
$Me^{II}O$ ausgewählt ist aus $MgO$, $CaO$, $SrO$, $ZnO$ und Mischungen davon,
$Me^{III}_2O_3$ ausgewählt ist aus $B_2O_3$, $Y_2O_3$, $La_2O_3$, $Ga_2O_3$, $In_2O_3$ und Mischungen davon,
$Me^{IV}O_2$ ausgewählt ist aus $TiO_2$, $ZrO_2$, $GeO_2$, $SnO_2$, $CeO_2$ und Mischungen davon,
$Me^V_2O_5$ ausgewählt ist aus $V_2O_5$, $Nb_2O_5$, $Ta_2O_5$ und Mischungen davon und
$Me^{VI}O_3$ ausgewählt ist aus $MoO_3$, $WO_3$ und Mischungen davon.

8. Glaskeramik nach einem der Ansprüche 1 bis 7, bei der das molare Verhältnis von $SiO_2$ zu $Li_2O$ im Bereich von 2,9 bis 4,6 und bevorzugt im Bereich von 3,3 bis 4,4 liegt.

9. Glaskeramik nach einem der Ansprüche 1 bis 8, die nicht mehr als 30 Gew.-%, bevorzugt nicht mehr als 28 Gew.-%, besonders bevorzugt nicht mehr als 26 Gew.-% und ganz besonders bevorzugt nicht mehr als 22 Gew.-% und insbesondere 10 bis 30 Gew.-%, bevorzugt 12 bis 28 Gew.-%, besonders bevorzugt 15 bis 26 Gew.-% und ganz besonders bevorzugt 18 bis 22 Gew.-% an Lithiummetasilikatkristallen enthält.

10. Glaskeramik nach einem der Ansprüche 1 bis 9, bei der die mittlere Größe der Lithiummetasilikatkristalle im Bereich von 5 bis 80 nm, insbesondere im Bereich von 10 bis 50 nm, bevorzugt im Bereich von 15 bis 45 nm und besonders bevorzugt im Bereich von 25 bis 35 nm liegt.

11. Ausgangsglas, das die Komponenten der Glaskeramik nach einem der Ansprüche 1 bis 8 enthält und insbesondere Keime für die Bildung von Lithiummetasilikatkristallen enthält.

12. Glaskeramik nach einem der Ansprüche 1 bis 10 oder Ausgangsglas nach Anspruch 11, wobei die Glaskeramik und das Ausgangsglas in Form von einem Pulver, einem Granulat, einem Rohling oder einer dentalen Restauration vorliegen.

13. Verfahren zur Herstellung der Glaskeramik gemäß einem der Ansprüche 1 bis 10 oder 12, bei dem das Ausgangsglas gemäß Anspruch 11 oder 12 mindestens einer Wärmebehandlung im Bereich von 450 bis 750°C unterzogen wird.

14. Verfahren nach Anspruch 13, bei dem

(a) das Ausgangsglas einer Wärmebehandlung bei einer Temperatur von 450 bis 600°C unterworfen wird, um Ausgangsglas mit Keimen zu bilden, und
(b) das Ausgangsglas mit Keimen einer Wärmebehandlung bei einer Temperatur von 550 bis 750°C unterworfen wird, um die Glaskeramik zu bilden.

15. Verwendung der Glaskeramik gemäß einem der Ansprüche 1 bis 10 oder 12 oder des Ausgangsglases gemäß Anspruch 11 oder 12 als Dentalmaterial, bevorzugt zur Beschichtung einer dentalen Restauration und besonders bevorzugt zur Herstellung einer dentalen Restauration.

16. Verwendung zur Herstellung einer dentalen Restauration nach Anspruch 15, wobei der Glaskeramik durch Verpressen oder maschinelle Bearbeitung die Form der gewünschten dentalen Restauration, insbesondere Brücke, Inlay, Onlay, Veneer, Abutment, Teilkrone, Krone oder Schale, gegeben wird.

17. Verwendung nach Anspruch 15 oder 16, bei der die Glaskeramik einer Wärmebehandlung bei einer Temperatur von 750 bis 950°C, bevorzugt 820 bis 890°C und besonders bevorzugt 840 bis 870°C, insbesondere für eine Dauer von 1 bis 60 Minuten, bevorzugt 5 bis 30 Minuten, besonders bevorzugt 5 bis 15 Minuten und ganz besonders bevorzugt 5 bis 10 Minuten, unterworfen wird.

18. Verfahren zur Herstellung einer dentalen Restauration, insbesondere Brücke, Inlay, Onlay, Veneer, Abutment, Teilkrone, Krone oder Schale, bei dem der Glaskeramik gemäß einem der Ansprüche 1 bis 10 oder 12 oder dem Ausgangsglas gemäß Anspruch 11 oder 12 durch Verpressen oder maschinelle Bearbeitung, insbesondere im Rahmen eines CAD/CAM-Verfahrens, die Form der gewünschten dentalen Restauration gegeben wird.

Europäisches
Patentamt

European
Patent Office

Office européen
des brevets

# EUROPÄISCHER RECHERCHENBERICHT

**Nummer der Anmeldung**

**EP 21 19 2546**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2015/104655 A1 (KIM YONG SU [KR] ET AL) 16. April 2015 (2015-04-16) * Absätze [0044], [0047] – [0050], [0059]; Tabelle 2 * ----- | 1-18 | INV. C03C3/097 C03C4/00 C03C10/00 |
| X | MARCUS P BOROM ET AL: "Strength and Microstructure in Lithium Disilicate Glass-Ceramics", CERAMIC ABSTRACTS,, Bd. 54, Nr. 9/10, 1. September 1975 (1975-09-01), Seiten 385-391, XP001261204, | 1,3,5-13 | |
| A | * Abschnitt III Results and Discussion, (1) Microstructure vs Heat Treatment, (A) Single-Stage Heat Treatment; Tabellen I and III * ----- | 14-18 | |
| X | BENGISU M ET AL: "Effect of long-term heating and thermal cycling on thermal expansion, phase distribution, and microhardness of lithium aluminosilicate glass-ceramics", JOURNAL OF NON-CRYSTALLINE SOLIDS, NORTH-HOLLAND PHYSICS PUBLISHING. AMSTERDAM, NL, Bd. 331, Nr. 1-3, 1. Dezember 2003 (2003-12-01), Seiten 137-144, XP004471757, ISSN: 0022-3093, DOI: 10.1016/J.JNONCRYSOL.2003.08.065 | 1,3,5-7, 10-13 | |
| A | * Tabellen 1,2 * ----- | 14-18 | |

**RECHERCHIERTE SACHGEBIETE (IPC)**

C03C

Der vorliegende Recherchenbericht wurde für alle Patentansprüche erstellt

| Recherchenort | Abschlußdatum der Recherche | Prüfer |
|---|---|---|
| München | 11. Februar 2022 | King, Ruth |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 21 19 2546

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-02-2022

| Im Recherchenbericht angeführtes Patentdokument | Datum der Veröffentlichung | Mitglied(er) der Patentfamilie | Datum der Veröffentlichung |
|---|---|---|---|
| US 2015104655 A1 | 16-04-2015 | EP 3059214 A1 | 24-08-2016 |
| | | KR 20150043633 A | 23-04-2015 |
| | | US 2015104655 A1 | 16-04-2015 |
| | | WO 2015056900 A1 | 23-04-2015 |

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9532678 A2
- EP 0827941 A1
- EP 0916625 A1
- EP 1505041 A1
- EP 1688398 A1
- EP 231773 A